(19) **Europäisches Patentamt**
**European Patent Office**
**Office européen des brevets**

(11) **EP 1 303 542 B3**

(12) **NOUVEAU FASCICULE DE BREVET EUROPEEN**
Après la procédure de limitation (B3-1)

(45) Date de publication et mention de la décision de limitation:
**B3-1 04.07.2012 Bulletin 2012/27**

(45) Mention de la délivrance du brevet:
**31.08.2005 Bulletin 2005/35**

(21) Numéro de dépôt: **01954103.6**

(22) Date de dépôt: **16.07.2001**

(51) Int Cl.:
*C08B 37/00* (2006.01)   *C08B 37/08* (2006.01)
*C08J 3/24* (2006.01)   *C08J 3/075* (2006.01)
*A61L 26/00* (2006.01)   *A61L 27/20* (2006.01)
*A61L 27/52* (2006.01)   *C08L 5/08* (2006.01)

(86) Numéro de dépôt international:
**PCT/FR2001/002300**

(87) Numéro de publication internationale:
**WO 2002/006350 (24.01.2002 Gazette 2002/04)**

(54) **PREPARATION D'ACIDE HYALURONIQUE RETICULE ET DE SON HYDROGEL, LES PRODUITS OBTENUS ET LEURS UTILISATIONS.**

HERSTELLUNG VON VERNETZTER HYALURONSÄURE UND DESSEN HYDROGEL, DIE HERGESTELLTEN PRODUKTE UND DEREN VERWENDUNGEN

PREPARATION OF CROSSLINKED HYALURONIC ACID AND THE HYDROGEL THEREFROM, THE PRODUCTS OBTAINED AND THE USES THEREOF

(84) Etats contractants désignés:
**DE ES FR GB IT**

(30) Priorité: **19.07.2000 FR 0009497**

(43) Date de publication de la demande:
**23.04.2003 Bulletin 2003/17**

(73) Titulaire: **Allergan Industrie**
**74370 Pringy (FR)**

(72) Inventeurs:
• **PIRON, Estelle**
  **31270 VILLENEUVE-TOLOSANE (FR)**

• **THOLIN, Raymonde**
  **F-74000 ANNECY (FR)**

(74) Mandataire: **Tetaz, Franck Claude Edouard et al**
**Cabinet Regimbeau**
**139, rue Vendôme**
**69477 Lyon Cedex 06 (FR)**

(56) Documents cités:
**EP-A- 0 161 887    CA-A- 949 965**

EP 1 303 542 B3

Printed by Jouve, 75001 PARIS (FR)

**Description**

La présente invention a pour objet :

- un procédé de préparation d'un hydrogel injectable d'au moins un polymère choisi parmi l'acide hyaluronique, ses sels et leurs mélange;
- les hydrogels injectables, susceptibles d'être obtenus par ces procédés ;
- un matériau de comblement, utile en chirurgie réparatrice ou en chirurgie esthétique, à base de tels hydrogels.

On a préparé, à partir de polysaccharides et de leurs dérivés - notamment à partir de l'acide hyaluronique, de ses sels et de leurs mélanges - pas, peu ou fortement réticulés, des hydrogels, notamment des hydrogels injectables. La demande de brevet EP-A-0 161 887 décrit ainsi l'utilisation de tels hydrogels injectables pour traiter l'arthrite. Les demandes de brevet WO-A-96/33751 et WO-A-00/01428 décrivent, quant à elles, des compositions biphasiques injectables dont la phase continue est à base d'un tel hydrogel. Ladite phase continue intervient à titre de véhicule d'injection.

D'après l'enseignement de ces documents (d'après l'enseignement de l'art antérieur, à la connaissance de la Demanderesse) la réticulation du polymère, et notamment celle de fibres de hyaluronate de sodium, est mise en oeuvre en présence d'un agent réticulant, notamment d'un polyépoxyde ; ledit agent réticulant intervenant, en quantité contrôlée, sur ledit polymère, dissous, en milieu basique. La réaction en cause est une réaction gel / liquide. Elle vise à générer un produit réticulé, aussi homogène que possible, précurseur d'un gel viscoélastique, facilement injectable, autant que faire se peut exempt de "paillettes", de surréticulations locales.

La présence de ces "paillettes" est fortement préjudiciable à l'injectabilité du gel en cause. Lors de son injection, des forces plus importantes sont nécessaires à appliquer lorsqu'une telle "paillette" se bloque dans l'aiguille d'injection. Ce problème est d'autant plus critique, que des aiguilles d'injection, de diamètres très fins, peuvent être utilisées.

C'est, en référence à ce problème technique de l'optimisation de l'injectabilité d'hydrogels, à base de tels polymères réticulés, que la présente invention a été développée.

Ladite présente invention propose en fait une mise en oeuvre originale de la réticulation, le polymère réticulé conduisant alors, après hydratation, à un hydrogel, très homogène, (quasi) exempt de surréticulations locales, qu'il est possible d'injecter avec une grande précision.

On a décrit, dans CA-A-949 965, une autre mise en oeuvre originale de réticulation, pour l'obtention de granules d'amidon. Selon celle-ci, on opère en trois principales étapes :

- une première étape d'hydratation de granules d'amidon au cours de laquelle lesdits granules se chargent en soude et en agent réticulant (épichlorhydrine) ;
- une seconde étape de récupération des granules chargés, par filtration ou centrifugation notamment ;
- une troisième étape de réticulation, par chauffage, desdits granules chargés récupérés.

Ladite réticulation est mise en oeuvre sur des granules, postérieurement à leur hydratation. Les granules obtenus sont homogènes dans leur masse mais leur ensemble ne constitue pas un grand volume parfaitement homogène dans sa masse.

Selon l'invention, en vue d'obtenir un polymère réticulé de façon très homogène dans sa masse, la réticulation est mise en oeuvre en même temps que l'hydratation, plus précisément au fur et à mesure que ladite hydratation prend place. Selon l'invention, on peut ainsi obtenir de grands volumes parfaitement homogènes dans leur masse (réticulés de manière isotrope).

La présente invention concerne donc un procédé de préparation d'un hydrogel injectable selon la revendication 1. De façon classique, la réticulation en cause est mise en oeuvre en présence d'au moins un agent réticulant polyfonctionnel (au moins bifonctionnel).

Selon l'invention, le procédé est caractérisé en ce que ledit agent réticulant polyfonctionnel est mis à réagir, directement, sur ledit polymère, à l'état solide, en cours d'hydratation.

Ledit polymère n'est donc pas, dans le cadre du procédé de l'invention, préalablement conditionné, au sens de l'art antérieur, par dissolution / gonflement.

De manière tout à fait surprenante, dans les conditions de l'invention, la réticulation est une réaction solide /liquide qui génère un polymère réticulé de façon homogène, polymère précurseur d'un hydrogel de structure parfaitement homogène.

De préférence, ledit agent réticulant polyfonctionnel intervient, sur ledit polymère solide, en solution basique. Ainsi, ledit agent réticulant polyfonctionnel peut être

- soit mis en solution ou en suspension dans une solution basique, cette solution ou suspension est ensuite mise à réagir directement sur ledit polymère à l'état solide,

- soit mis à réagir directement sur ledit polymère à l'état solide, l'ajout de l'agent basique se faisant simultanément à l'ajout de l'agent réticulant mais par moyen d'introduction séparé, sur ledit polymère à l'état solide.

Généralement, lorsque ledit agent réticulant est en solution basique, cette solution basique a un pH compris entre 10 et 12.

A titre d'agent réticulant, on peut faire intervenir tout agent connu pour réticuler les polysaccharides et leurs dérivés, par l'intermédiaire de ses fonctions hydroxy - agent réticulant au moins bifonctionnel - et notamment un époxyde ou ses dérivés.

A titre de tels agents réticulants, on peut notamment faire intervenir le 1,4-butanedioldiglycidyléther(ou 1,4-bis (2,3-époxypropoxybutane ou encore 1,4-bisglycidyloxybutane = BDDE), le 1,2bis(2,3-époxypropoxy)éthylène et le 1-(2,3-époxypropyl)-2,3-époxycyclohexane.

Il n'est pas exclu du cadre de l'invention de faire intervenir plusieurs agents réticulants.

On préconise tout particulièrement de faire intervenir le 1,4-butanedioldiglycidyléther (BDDE).

Ledit polymère en cause (les polymères en cause) est(sont) choisi(s) parmi l'acide hyaluronique, ses sels et leurs mélanges.

On préconise tout particulièrement le hyaluronate de sodium.

La réticulation à mettre en oeuvre sur ledit polymère est à la portée de l'homme du métier. Pour chaque polymère l'homme du métier saura optimiser les conditions de cette réticulation à la nature dudit polymère et la réaliser à un taux optimisé.

En effet, le taux de réticulation doit être suffisant pour que l'hydrogel, obtenu à partir de ce polymère réticulé, reste implanté au site d'injection sans diffusion excessive hors de ce site d'injection ; il doit toutefois rester raisonnable pour une utilisation, en particulier, par injection.

Cependant, de manière préférée, selon l'invention, le polymère est réticulé, *via* ses fonctions hydroxy, au moyen dudit agent réticulant à un taux de réticulation défini par le rapport :

$$R = \frac{\text{Nombre total de fonctions réactives dudit agent réticulant}}{\text{Nombre total de motifs disaccharides des molécules d'acide hyaluronique}}$$

compris entre 0,10 et 0,50.

L'invention concerne un procédé de préparation d'un hydrogel injectable d'au moins un

L'invention concerne un procédé de préparation d'un hydrogel injectable d'au moins un polymère réticulé choisi parmi l'acide hyaluronique, ses sels et leurs mélanges.

De façon caractéristique, ce procédé comprend la réticulation dudit polymère selon le procédé de réticulation de l'invention décrit ci-dessus, dans toutes ses variantes, ladite réticulation étant suivie d'un gonflement dudit polymère réticulé, par hydratation.

Le procédé de préparation d'un hydrogel injectable de l'invention est optimisé en référence à la présence de réticulations ou de surréticulations locales au sein dudit hydrogel.

Ledit procédé de préparation dudit hydrogel est par ailleurs *per se* optimisé en référence à sa durée de mise en oeuvre.

Selon l'invention, il est ainsi possible d'obtenir en 2 à 3 heures un hydrogel réticulé par une réaction en phase solide/liquide alors que selon l'art antérieur un hydrogel réticulé est obtenu en environ 5 h, par une réaction en phase gel/liquide.

Ce gain de temps est appréciable *per se.* Il permet en effet l'obtention d'une meilleure qualité du produit final ; la dégradation du polymère, qui se produit en particulier en milieu basique, étant alors limitée. De plus, on garantit une meilleure reproductibilité de la synthèse des gels obtenus en final.

Ledit procédé comprend, de façon caractéristique, les étapes successives ci-après :

- la réticulation d'un polymère ou d'un mélange de polymères, choisi(s) parmi les polysaccharides et leurs dérivés, selon le procédé de réticulation précédemment décrit,
- le gonflement dudit(desdits) polymère(s) réticulé(s),
- la purification dudit (desdits) polymère(s) réticulé(s), gonflé(s),
- la stérilisation, si nécessaire, dudit (desdits) polymère(s) réticulé(s), gonflé(s), purifié(s).

Les étapes de réticulation, de purification et de stérilisation sont des étapes *per se* connues de l'homme du métier. Ladite purification est nécessaire pour éliminer les produits n'ayant pas ou incomplètement réagis tels le(s) polymère(s) et le(s) agent(s) réticulant(s). Ladite étape de stérilisation est évidemment nécessaire si les étapes en amont n'ont pas été mises en oeuvre dans des conditions stériles.

Un autre objet de l'invention est hydrogel injectable susceptible d'être obtenu à l'issue de la mise en oeuvre dudit procédé de préparation d'un hydrogel injectable selon l'invention tel que décrit précédemment, dans ses différentes variantes. Cet hydrogel est caractérisé par la faible présence, voire l'absence, de surréticulations locales.

L'hydrogel injectable, tel que décrit ci-dessus, tel qu'obtenu par le procédé ci-dessus, convient parfaitement pour la fabrication d'un gel de comblement, utile en chirurgie réparatrice ou esthétique.

Un autre objet de l'invention est donc un gel de comblement utile en chirurgie réparatrice ou esthétique qui est à base d'un hydrogel selon l'invention et/ou tel qu'obtenu selon le procédé de l'invention.

L'hydrogel de l'invention constitue au moins la phase continue dudit gel de comblement de l'invention.

Ainsi ledit gel de comblement peut être constitué d'une seule phase qui est l'hydrogel réticulé de l'invention.

Il peut également être bi- ou pluri-phasique c'est-à-dire qu'il peut être constitué de plusieurs phases dont au moins la phase continue est l'hydrogel réticulé de l'invention.

La seconde et les autres phases peuvent être par exemple des billes elles-mêmes constituées de l'hydrogel réticulé de l'invention mais, dans ce cas, l'hydrogel constituant les billes présente un taux de réticulation plus élevé que le taux de réticulation de l'hydrogel réticulé constituant la phase continue du gel de comblement de l'invention. Les billes peuvent également être en acrylique ou polymère acrylique.

De préférence, le gel de comblement selon l'invention est constitué d'un hydrogel réticulé selon l'invention. Il est monophasique.

Le(s) hydrogel(s) plus ou moins réticulé(s) formant la base du gel de comblement de l'invention peut(peuvent) indifféremment être chargé(s) ou non chargé(s). Ils peuvent, par exemple, être chargés avec des antiseptiques et éventuellement d'autres substances, autres substances dont l'intervention est avantageuse dans les zones du corps humain ou animal concerné.

Ainsi ledit hydrogel constituant au moins la phase continue d'un gel de comblement de l'invention renferme avantageusement de l'acide désoxyribonucléique (ADN). Ce produit est connu pour diminuer la réaction inflammatoire et favoriser la régénération tissulaire.

Le gel de comblement de l'invention présente l'avantage d'être facilement injectable, c'est-à-dire injectable au travers d'aiguilles très fines de 26G à 30G, sans nécessiter de pics de pression, en raison du taux de réticulation homogène au travers de toute la masse de l'hydrogel réticulé utilisé et qui est obtenu par le procédé de l'invention.

Cette homogénéité de réticulation conjuguée au taux de réticulation résulte de l'absence ou la quasi absence de "paillettes" plus réticulée dans le gel, et ce gel peut alors être injecté avec une force constante et ainsi avec une meilleure précision, au site voulu.

Le gel de comblement de l'invention peut être utilisé en chirurgie esthétique, par exemple, pour le comblement des rides d'une manière plus précise, plus fiable et plus aisée car il a un niveau d'injectabilité très régulier, en contraste aux gels de comblements de l'art antérieur.

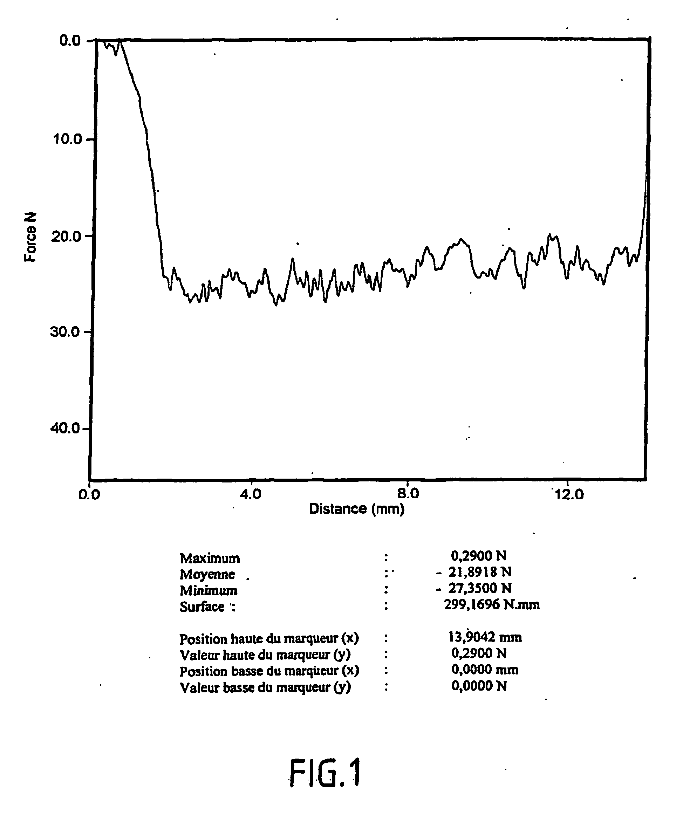

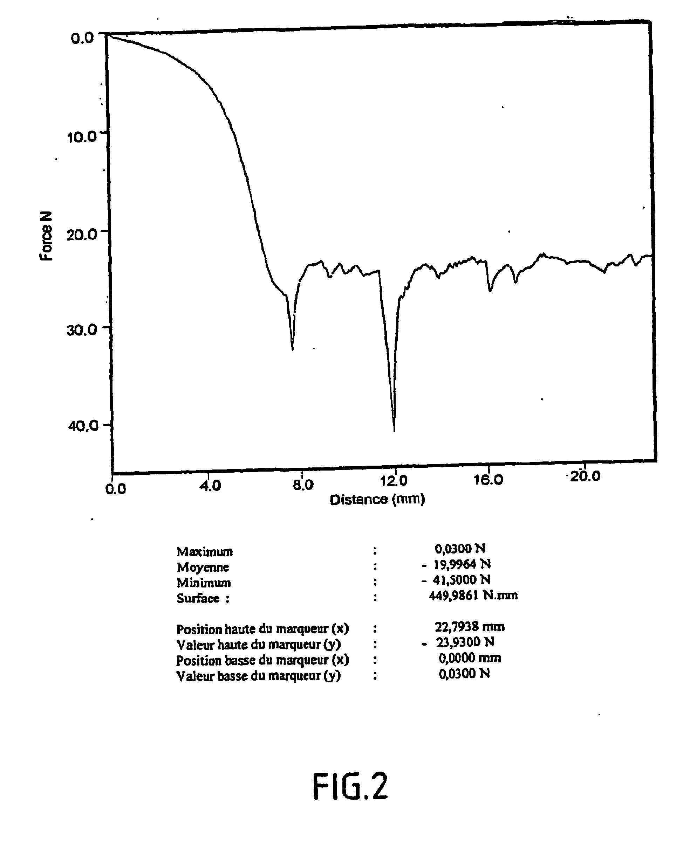

D'autres buts, caractéristiques et avantages de l'invention sont maintenant illustrés par les exemples ci-après décrivant, en référence aux figures 1 et 2 annexées, la préparation d'un gel de comblement préféré selon l'invention, la préparation d'un gel de comblement de l'art antérieur et les tests d'injectabilités réalisés sur ces gels.

Dans les figures :

- la figure 1 montre la courbe d'injectabilité du gel de comblement selon l'invention préparé à l'exemple 1, c'est-à-dire la force nécessaire pour injecter ce gel en fonction de la durée d'injection,
- la figure 2 montre la courbe d'injectabilité du gel de comblement selon l'art antérieur préparé à l'exemple 2, c'est-à-dire la force nécessaire pou injecter ce gel en fonction de la durée d'injection.

Exemple 1 : Préparation d'un gel de comblement selon l'invention

- On pèse 1 g de fibres de hyaluronate de sodium (NaHa de masse moléculaire $M_w \approx 2.10^6$ Da) préalablement séchées, dans un premier récipient.
- Dans un récipient séparé, on dilue l'agent réticulant choisi, le 1,4-butanedioldiglycidyléther (BDDE), dans de la soude à 1 % pour obtenir une solution basique de BDDE diluée au 100$^{éme}$.
- On ajoute alors 6,8 g de la solution de BDDE diluée au 100$^{éme}$ précédemment préparée sur les fibres, toujours à l'état solide, de NaHa, et on procède à une homogénéisation mécanique du mélange, à la spatule.

- Le mélange est ensuite placé dans un bain marie à 50°C $\pm$ 2°C pendant 2 à 3 heures avec une nouvelle homogénéisation après 15 minutes d'immersion, pour provoquer la réticulation du polymère de NaHa.
- Le polymère de NaHa réticulé obtenu est ensuite immergé dans un tampon phosphate (TP) pour stabiliser le pH.

Cette étape correspond à l'étape de gonflement par hydratation du polymère de NaHa réticulé menant à l'obtention de l'hydrogel de l'invention.

- Le polymère de NaHa, réticulé, gonflé est ensuite purifié par immersion dans différents bains de tampon phosphate afin d'éliminer l'agent réticulant et le NaHa qui n'ont pas réagi.
- L'hydrogel obtenu est alors homogénéisé mécaniquement pour assurer l'homogénéité finale et conditionné dans des seringues qui sont stérilisées à l'autoclave.

Comme on le voit dans cet exemple, le gel de comblement de l'invention est constitué uniquement de l'hydrogel obtenu ou susceptible d'être obtenu par le procédé de l'invention.

Le gel obtenu est injectable avec une canule de 27G1/2 ou 30G1/2 pour une augmentation des volumes en sous cutané.

Exemple 2 : Préparation d'un gel de comblement selon le procédé de l'art antérieur

- On pèse 1 g de fibres de hyaluronate de sodium (NaHa, de masse moléculaire $M_w \approx 2.10^6$ Da), préalablement séchées, dans un récipient.
- On ajoute dans le même récipient contenant les fibres de NaHa, 6,7 g de soude à 1 % et on laisse reposer à température ambiante pendant 2 h 30 à 3 h 00 avec homogénéisation toutes les ½ heures.

Cette étape correspond à l'étape d'hydratation des fibres de NaHa. On obtient le polymère de NaHa non réticulé, sous forme de gel.

- On ajoute ensuite 60 mg du même agent réticulant que celui utilisé à l'exemple 1, le BDDE, dans le même récipient contenant le gel de NaHa et la soude et on procède à une homogénéisation mécanique du BDDE dans le gel de NaHa.

- Le mélange est ensuite placé dans un bain marie à 50°C $\pm$ 2°C pendant 1 h 45 à 2 h 30.

Cette étape correspond à l'étape de réticulation du polymère de NaHa.

- Le polymère de NaHa réticulé ainsi obtenu est ensuite immergé dans un tampon phosphate (TP) pour stabiliser le pH. Cette étape correspond à l'étape de gonflement par hydratation du polymère réticulé.
- Le polymère de NaHa, réticulé, gonflé est ensuite purifié par immersion dans différents bains de tampon phosphate afin d'éliminer l'agent réticulant et le NaHa qui n'auraient pas réagis.

L'hydrogel obtenu est alors homogénéisé mécaniquement pour assurer l'homogénéité finale conditionnée dans des seringues qui sont stérilisées à l'autoclave.

Dans cette exemple également, le gel de comblement est constitué uniquement de l'hydrogel réticulé, gonflé, purifié et stabilisé obtenu avec le procédé de l'art antérieur.

Exemple 3 : Tests d'injectabilité des gels de comblement obtenus

Afin de comparer l'injectabilité du gel de comblement obtenu selon le procédé de l'invention à celle du gel de comblement obtenu selon le procédé de l'art antérieur, des essais d'injectabilité ont été effectués sur chacun d'eux.

Ces essais d'injectabilité ont été effectués sur un appareil de traction Versatet (commercialisé par Mecmesin). Les gels ont été chacun injectés à travers une aiguille de 27G1/2 à une vitesse de compression de 12,5 mm/mn. La force nécessaire, exprimée en Newton, pour injecter les gels est caractéristique du taux de réticulation et de la concentration en NaHa du gel, et la régularité de cette force est caractéristique de la facilité d'injection du gel ainsi que de son homogénéité.

En effet, lorsque le gel comprend des paillettes plus réticulées, la force nécessaire pour injecter le gel augmente puis diminue lorsque la paillette a traversé l'aiguille, ce qui se traduit par des pics au niveau de la force nécessaire pour maintenir la vitesse d'injection de 12,5 mm/mn.

Les résultats des mesures effectuées sur le gel de comblement de l'invention sont montrés sur le graphe de la figure 1 et les résultats des mesures effectués sur le gel de comblement fabriqué selon le procédé de l'art antérieur sont montrés sur le graphe de la figure 2.

Sur ces graphes, la force nécessaire en Newton (N) pour injecter le gel, est représentée en ordonnées et le temps d'injection, représenté par la longueur en mm de déroulement du papier, est représenté en abscisse. Il est à noter que sur ces figures, une augmentation de la force nécessaire pour injecter le gel de comblement se traduit par un pic descendant par rapport à la ligne de base.

Comme on le voit sur les figures 1 et 2, la force nécessaire pour injecter le gel de comblement selon l'invention est beaucoup plus régulière: la force nécessaire est d'environ 27 Newton, en moyenne, sans présence de pics dus à la présence de paillettes. En contraste, bien que la force moyenne pour injecter le gel de comblement fabriqué selon le procédé de l'art antérieur soit également d'environ 27 Newton, 2 pics importants d'une amplitude de 30 à 42 Newton apparaissent lors de l'injection du gel selon l'art antérieur. Ces pics sont caractéristiques de surréticulations locales.

Ainsi, le gel obtenu par le procédé de l'invention présente une réticulation plus homogène que le gel fabriqué selon le procédé de l'art antérieur.

**Revendications**

1. Procédé de préparation d'un hydrogel injectable d'au moins un polymère réticulé choisi parmi l'acide hyaluronique, ses sels et leurs mélanges, **caractérisé en ce qu'**il comprend la réticulation dudit polymère sous l'action d'au moins un agent réticulant polyfonctionnel, dans laquelle ledit agent réticulant polyfonctionnel est mis à réagir sur ledit polymère, à l'état solide, en cours d'hydratation ; suivie d'un gonflement dudit polymère réticulé par hydratation.

2. Procédé de préparation d'un hydrogel injectable selon la revendication 1, **caractérisé en ce qu'**il comprend :

   - la réticulation dudit polymère sous l'action d'au moins un agent réticulant polyfonctionnel, dans laquelle ledit agent réticulant polyfonctionnel est mis à réagir sur ledit polymère, à l'état solide, en cours d'hydratation ;
   - le gonflement dudit polymère réticulé ;
   - la purification dudit polymère réticulé gonflé ;
   - la stérilisation, si nécessaire, dudit polymère réticulé gonflé purifié.

3. Procédé de préparation d'un hydrogel injectable selon l'une des revendications 1 ou 2, **caractérisé en ce que** ledit agent réticulant polyfonctionnel intervient, sur ledit polymère solide, en solution basique.

4. Procédé de préparation d'un hydrogel selon l'une quelconque des revendications 1-3, **caractérisé en ce que** ledit agent réticulant polyfonctionnel est un époxyde.

5. Procédé de préparation d'un hydrogel injectable selon la revendication 4 **caractérisé en ce que** ledit agent réticulant polyfonctionnel est choisi parmi le 1,4-butanedioldiglycidylether, le 1,2bis(2,3-époxypropoxy)éthylène et le 1-(2,3-époxypropyl)-2,3-époxycyclohexane.

6. Procédé de préparation d'un hydrogel injectable selon la revendication 5 **caractérisé en ce que** ledit agent réticulant polyfonctionnel consiste en le 1,4-butanedioldiglycidyléther.

7. Procédé de préparation d'un hydrogel injectable selon l'une quelconque des revendications 1 à 6, **caractérisé en ce que** ledit polymère consiste en le hyaluronate de sodium.

8. Procédé de préparation d'un hydrogel injectable selon l'une quelconque des revendications 1 à 7, **caractérisé en ce que** ledit polymère est réticulé, *via* ses fonctions hydroxy, au moyen dudit agent réticulant à un taux de réticulation défini par le rapport :

$$R = \frac{\text{Nombre total de fonctions réactives dudit agent réticulant}}{\text{Nombre total de motifs disaccharides des molécules d'acide hyaluronique}}$$

compris entre 0,10 et 0,50.

9. Hydrogel injectable susceptible d'être obtenu à l'issue de la mise en oeuvre d'un procédé de préparation selon l'une des revendications 1 à 8.

10. Gel de comblement, utile en chirurgie réparatrice ou esthétique, **caractérisé en ce qu'**il est à base d'un hydrogel, chargé ou non, selon la revendication 9.

**11.** Gel de comblement selon la revendication 10, **caractérisé en ce qu'**il consiste en un hydrogel, monophasique, selon la revendication 9.

**Claims**

**1.** A process for the preparation of an injectable hydrogel of at least one crosslinked polymer selected from hyaluronic acid, its salts and their mixtures, **characterized in that** it comprises the crosslinking of said polymer under the action of at least one polyfunctional crosslinking agent, wherein said polyfunctional crosslinking agent is reacted with said polymer, in the solid state, during hydration; followed by the swelling of said crosslinked polymer by hydration.

**2.** A process for the preparation of an injectable hydrogel according to claim 1, **characterized in that** it comprises:

- the crosslinking of said polymer under the action of at least one polyfunctional crosslinking agent, wherein said polyfunctional crosslinking agent is reacted with said polymer, in the solid state, during hydration;
- the swelling of said crosslinked polymer;
- the purification of said swollen crosslinked polymer; and
- the sterilization, if necessary, of said purified swollen crosslinked polymer.

**3.** A process for the preparation of an injectable hydrogel according to anyone of claims 1-2, **characterized in that** said polyfunctional crosslinking agent reacts with said solid polymer in basic solution.

**4.** A process for the preparation of an injectable hydrogel according to anyone of claims 1-3, **characterized in that** said polyfunctional crosslinking agent is an epoxide.

**5.** A process for the preparation of an injectable hydrogel according to claim 4, **characterized in that** said polyfunctional crosslinking agent is selected from 1,4-butanediol diglycidyl ether, 1,2-bis(2,3-epoxypropoxy)ethylene and 1-(2,3-epoxypropyl)-2,3-epoxycyclo-hexane.

**6.** A process for the preparation of an injectable hydrogel according to claim 5, **characterized in that** said polyfunctional crosslinking agent consists of 1,4-butanediol diglycidyl ether.

**7.** A process for the preparation of an injectable hydrogel according to any one of claims 1 to 6, **characterized in that** said polymer consists of sodium hyaluronate.

**8.** A process for the preparation of an injectable hydrogel according to any one of claims 1 to 7, **characterized in that** said polymer is crosslinked, via its hydroxyl groups, by means of said crosslinking agent to a degree of crosslinking defined by a ratio

$$R = \frac{\text{Total number of reactive groups in said crosslinking agent}}{\text{Total number of disaccharide units in the hyaluronic acid molecules}}$$

of between 0.10 and 0.50.

**9.** An injectable hydrogel obtainable by carrying out a preparative process according to anyone of claims 1-8.

**10.** A filling gel useful in plastic or cosmetic surgery, **characterized in that** it is based on a charged or uncharged hydrogel according to claim 9.

**11.** The filling gel according to claim 10, **characterized in that** it consists of a monophase hydrogel according to claim 9.

**Patentansprüche**

1. Verfahren zur Herstellung eines spritzbaren Hydrogels aus wenigstens einem vernetzten Polymeren, ausgewählt aus Hyaluronsäure, deren Salzen und deren Mischungen, **dadurch gekennzeichnet, dass** es die Vernetzung des Polymeren unter Einwirkung wenigstens eines polyfunktionellen Vernetzungsmittels umfasst, in der das polyfunktionelle Vernetzungsmittel auf das Polymere in festem Zustand im Verlauf der Hydratation einwirkt, und ein anschliessendes Aufquellen des vernetzten Polymeren durch Hydratation umfasst.

2. Verfahren zur Herstellung eines spritzbaren Hydrogels gemäss Anspruch 1, **dadurch gekennzeichnet, dass** es:

    - die Vernetzung des Polymeren unter Einwirkung wenigstens eines polyfunktionellen Vernetzungsmittels, in der das polyfunktionelle Vernetzungsmittel auf das Polymer in festem Zustand im Verlauf der Hydratation einwirkt,
    - das Aufquellen des vernetzten Polymeren,
    - die Reinigung des gequollenen, vernetzten Polymeren und, gegebenenfalls,
    - die Sterilisation des gereinigten, gequollenen, vernetzten Polymeren umfasst.

3. Verfahren zur Herstellung eines spritzbaren Hydrogels gemäss einem der Ansprüche 1 oder 2, **dadurch gekennzeichnet, dass** das polyfunktionelle Vernetzungsmittel auf das feste Polymere in basischer Lösung einwirkt.

4. Verfahren zur Herstellung eines spritzbaren Hydrogels gemäss einem der Ansprüche 1 bis 3, **dadurch gekennzeichnet, dass** das polyfunktionelle Vernetzungsmittel ein Epoxid ist.

5. Verfahren zur Herstellung eines spritzbaren Hydrogels gemäss Anspruch 4, **dadurch gekennzeichnet, dass** das polyfunktionelle Vernetzungsmittel aus 1,4-Butandioldiglycidylether, 1,2-Bis(2,3-epoxypropoxy)ethylen und 1-(2,3-Epoxypropyl)-2,3-epoxycyclohexan ausgewählt wird.

6. Verfahren zur Herstellung eines spritzbaren Hydrogels gemäss Anspruch 5, **dadurch gekennzeichnet, dass** es sich bei dem polyfunktionellen Vernetzungsmittel um 1,4-Butandioldiglycidylether handelt.

7. Verfahren zur Herstellung eines spritzbaren Hydrogels gemäss einem der Ansprüche 1 bis 6, **dadurch gekennzeichnet, dass** es sich bei dem Polymeren um Natriumhyaluronat handelt.

8. Verfahren zur Herstellung eines spritzbaren Hydrogels gemäss einem der Ansprüche 1 bis 7, **dadurch gekennzeichnet, dass** das Polymere über seine Hydroxygruppen mit Hilfe des Vernetzungsmittels mit einem Vernetzungsgrad vernetzt ist, der durch ein Verhältnis:

$$R = \frac{\text{Gesamtzahl an reaktiven Gruppen des Vernetzungsmittels}}{\text{Gesamtzahl an Disaccharideinheiten der Hyaluronsäuremoleküle}}$$

von 0,10 bis 0,50 definiert ist.

9. Spritzbares Hydrogel, welches durch ein Herstellungsverfahren gemäss irgend einem der Ansprüche 1 bis 8 erhältlich ist.

10. Füllgel, welches in der reparierenden oder ästhetischen Chirurgie verwendet werden kann, **dadurch gekennzeichnet, dass** es auf einem, gegebenenfalls beladenen, Hydrogel gemäss Anspruch 9 basiert.

11. Füllgel gemäss Anspruch 10, **dadurch gekennzeichnet, dass** es aus einem einphasigen Hydrogel gemäss Anspruch 9 besteht.

| Maximum | : | 0,2900 N |
| Moyenne . | : · | - 21,8918 N |
| Minimum | : | - 27,3500 N |
| Surface : | : | 299,1696 N.mm |
| | | |
| Position haute du marqueur (x) | : | 13,9042 mm |
| Valeur haute du marqueur (y) | : | 0,2900 N |
| Position basse du marqueur (x) | : | 0,0000 mm |
| Valeur basse du marqueur (y) | : | 0,0000 N |

# FIG.1

| Maximum | : | 0,0300 N |
|---|---|---|
| Moyenne | : | - 19,9964 N |
| Minimum | : | - 41,5000 N |
| Surface : | : | 449,9861 N.mm |
| | | |
| Position haute du marqueur (x) | : | 22,7938 mm |
| Valeur haute du marqueur (y) | : | - 23,9300 N |
| Position basse du marqueur (x) | : | 0,0000 mm |
| Valeur basse du marqueur (y) | : | 0,0300 N |

# FIG.2

**RÉFÉRENCES CITÉES DANS LA DESCRIPTION**

**Documents brevets cités dans la description**

- EP 0161887 A
- WO 9633751 A
- WO 0001428 A
- CA 949965 A